# FASCICULE DE BREVET EUROPEEN

(11) **EP 4 262 552 B1**
(45) Date de publication et mention de la délivrance du brevet: **29.10.2025**
(21) Numéro de dépôt: 21823324.5
(22) Date de dépôt: 08.12.2021
(51) Int. Cl.: A61B 5/11, A61B 5/055, A61B 5/245, A61B 5/243, A61B 5/00

(54) **EQUIPEMENT POUR L'AMELIORATION DE LA PRECISION D'UNE IMAGE BIOMAGNETIQUE D'UN PATIENT**
VORRICHTUNG ZUR VERBESSERUNG DER PRÄZISION EINES BIOMAGNETISCHEN BILDES EINES PATIENTEN
DEVICE FOR IMPROVING THE PRECISION OF A BIOMAGNETIC IMAGE OF A PATIENT

(30) Priorité: 17.12.2020 FR 2013422
(43) Date de publication de la demande: 25.10.2023
(73) Titulaire: Commissariat à l'Energie Atomique et aux Energies Alternatives, 75015 Paris (FR)
(72) Inventeur: LABYT, Etienne, 38054 Grenoble Cedex 09 (FR)
(74) Mandataire: Atout PI Laplace
(86) Numéro de dépôt international: PCT/EP2021/084788
(87) Numéro de publication internationale: WO 2022/128683

(56) Documents cités:
- US-A1- 2017 352 457
- US-A1- 2017 352 457
- PFEIFFER CHRISTOPH ET AL: "On-scalp MEG sensor localization using magnetic dipole-like coils: A method for highly accurate co-registration", NEUROIMAGE, ELSEVIER, AMSTERDAM, NL, vol. 212, 28 February 2020 (2020-02-28), XP086128806, ISSN: 1053-8119, [retrieved on 20200228], DOI: 10.1016/J.NEUROIMAGE.2020.116686

## Description

### Domaine technique

La présente invention concerne un équipement pour l'amélioration de la précision d'une image biomagnétique ainsi qu'un procédé associé.

### Technique antérieure

Plusieurs techniques d'imagerie médicale existent aujourd'hui pour obtenir des informations sur une partie d'un corps d'un patient.

L'imagerie par résonnance magnétique dit IRM est une technique d'imagerie médicale permettant d'obtenir des vues en deux ou trois dimensions de l'intérieur du corps de façon non invasive avec une résolution en contraste relativement élevée. Le principe de l'IRM repose sur le phénomène de résonance magnétique nucléaire portant sur le couplage entre le moment magnétique d'un noyau d'un atome et le champ magnétique externe. L'IRM nécessite un champ magnétique puissant et stable produit par un aimant supraconducteur qui crée une magnétisation des tissus par alignement des moments magnétiques de spin. Des champs magnétiques oscillants plus faibles, dits radiofréquence, sont alors appliqués de façon à légèrement modifier cet alignement et produire un phénomène de précession qui donne lieu à un signal magnétique mesurable. L'IRM est principalement dédiée à l'imagerie des tissus mous (cerveau, muscles, cœur, poumon et viscères) et des tumeurs.

L'imagerie biomagnétique est une technique d'imagerie des champs magnétiques générés par le corps humain. Elle permet ainsi de mesurer des champs magnétiques induits par l'activité électrique de différentes parties du corps en vue d'obtenir des données biomagnétiques. Dans l'imagerie biomagnétique, on peut distinguer la magnétoencéphalographie dite MEG. Cette technique MEG est employée avec une visée clinique en neurologie ainsi que dans la recherche en neurosciences cognitives et elle fournit des données biomagnétiques de type données MEG. Dans l'imagerie biomagnétique, on distingue également la magnétocardiographie dite MCG. Cette technique MCG est utilisée en cardiologie. Les champs magnétiques mesurés étant extrêmement faibles, la MEG et la MCG utilisent un appareillage basé sur des capteurs SQUID (acronyme de « Superconducting Quantum Interference Device » en anglais) refroidis par un fluide cryogénique au sein d'un Dewar. Dans le cas d'une analyse portant sur le cerveau, les capteurs SQUID sont positionnés à quelques centimètres de la tête du patient et sont figés dans cette position. Cet éloignement des capteurs, imposé par l'utilisation du fluide cryogénique, altère la qualité du signal mesuré. La tête du patient peut également bouger par rapport à ces capteurs, ce qui altère aussi la précision de l'imagerie réalisée par la MEG. Afin de résoudre ce problème, des travaux de recherche sont effectués pour réaliser une magnétoencéphalographie à partir de magnétomètres à pompage optique. L'ensemble des recherches dans ce domaine se base sur les magnétomètres à pompage optique d'atomes alcalins. Il existe une autre catégorie de magnétomètres à pompage optique basés sur de l'hélium. Il est connu un casque support permettant l'ajustement de la position de chaque magnétomètre au plus proche de la tête du patient. Dans le cas des magnétomètres à pompage optique de l'hélium, une procédure d'auto-localisation de ces magnétomètres fournit directement les positions et les orientations desdits magnétomètres les uns par rapport aux autres sur la tête du patient. Le document FR3056761 divulgue une méthode de calibration des différents magnétomètres à pompage optique de l'hélium entre eux pour leur permettre une auto-localisation.

Le document US2017/352457A1 divulgue une bobine de marquage utilisée avec un magnétoencéphalographe.

Afin de localiser les régions actives du corps du patient à l'origine d'une activité électrique, telles que le cerveau, il est nécessaire de fusionner des données issues de la magnétoencéphalographie avec des données anatomiques issues de l'imagerie par résonnance magnétique pour obtenir une image combinée. Ceci se fait en recalant la position des capteurs MEG (SQUID ou magnétomètres à pompage optique alcalins) dans le système de coordonnées dans lequel est référencé le volume reconstruit à partir de l'IRM. Actuellement, la fusion des données MEG avec les données IRM se fait en deux étapes. Dans une première étape, il est procédé à une localisation de points singuliers tels que des saillies osseuses, le nasion, les points pré-auriculaires droit et gauche, etc... à l'aide d'un système externe optique ou électromagnétique au moment des enregistrements des données MEG. Ceci permet de recaler la position des capteurs MEG par rapport à la tête du patient. Dans une seconde étape, il est procédé à une reconstruction 3D de l'anatomie avec un repérage des mêmes points singuliers sur les images IRM. Les points singuliers sont ainsi localisés par deux moyens différents en IRM et en MEG.

Cependant, l'étape de recalage de la position des magnétomètres par rapport à la position de la tête et l'étape de fusion de données MEG et IRM sont toutes deux sources de biais qui altèrent la précision de localisation fonctionnelle en MEG.

Il existe donc un besoin d'améliorer cette procédure de recalage et fusion des données MEG/IRM au moyen d'un dispositif permettant l'obtention d'une imagerie médicale de plus grande précision.

### Exposé de l'invention

La présente invention vise à répondre à ce besoin.

Plus particulièrement, la présente invention propose un équipement qui vise à améliorer la procédure de recalage/fusion des données MEG/IRM ou MCG/IRM pour l'obtention d'une image médicale présentant une plus grande précision.

Un premier objet de l'invention concerne un équipement pour l'amélioration de la précision d'une image biomagnétique d'un patient. Cet équipement comporte une enveloppe adaptée pour être positionnée sur une partie anatomique du patient. L'équipement comporte une pluralité de marqueurs disposés sur l'enveloppe, lesdits marqueurs étant adaptés pour créer un contraste lors d'une obtention d'une image du patient réalisée par imagerie par résonnance magnétique. L'équipement comporte également au moins cinq bobines trois axes, chaque bobine trois axes étant adaptée pour émettre un champ magnétique, les champs magnétiques desdites bobines trois axes étant détectables par un ensemble de magnétomètres à pompage optique lors d'un examen par imagerie biomagnétique. Ces bobines trois axes sont disposées sur l'enveloppe de sorte que lorsque l'équipement est positionné sur le patient, chacune de ces bobines trois axes est localisée sur un point singulier du patient. En outre, les bobines trois axes et des marqueurs de la pluralité de marqueurs sont placés au même endroit sur l'enveloppe de sorte que lorsque ladite enveloppe est positionnée sur le patient, des points singuliers de la partie du patient sont détectables par imagerie par résonnance magnétique et par imagerie biomagnétique. L'équipement comporte des supports, chaque support ayant une première zone de réception adaptée pour recevoir un des marqueurs et une seconde zone de réception adaptée pour recevoir une des bobines trois axes. Le marqueur et la bobine trois axes sont alors placés au même endroit sur l'enveloppe. La seconde zone de réception comprend une protubérance adaptée pour venir se loger dans une cavité associée de la bobine trois axes. La protubérance comprend un corps principal et un pion s'étendant à partir dudit corps principal. La protubérance a un rôle de détrompeur pour le positionnement de la bobine trois axes dans le support.

L'équipement est conçu pour être porté par le patient dans les deux modalités d'imagerie : biomagnétique et IRM. Il facilite donc l'exploitation des données obtenues par ces deux modalités d'imagerie médicale. Les bobines trois axes sont co-localisées avec des marqueurs sur l'enveloppe sur des mêmes points singuliers. Cela permet d'assurer un recalage optimum des données MEG/IRM ou des données MCG/IRM en vue d'obtenir une image médicale combinée présentant une grande précision.

Les bobines trois axes et les marqueurs sont positionnés dans un support commun accroché à l'enveloppe de l'équipement. On réalise ainsi de manière précise, simple et pratique la co-localication de ces bobines et de ces marqueurs, au niveau des points singuliers sur le patient.

On maintient également efficacement la bobine trois axes dans le support de l'enveloppe.

On assure en outre une orientation précise et reproductible des trois axes de la bobine.

Dans un mode de réalisation particulier, l'enveloppe est réalisée dans un matériau en silicone biocompatible souple.

Dans un mode de réalisation particulier, le marqueur comprend du gadolinium et/ou une pluralité de nanoparticules superparamagnétiques.

Dans un mode de réalisation particulier, chaque marqueur a la forme d'une pastille présentant un diamètre inférieur ou égal à 6 mm.

Dans un mode de réalisation particulier, l'équipement est positionné sur un visage du patient ou l'équipement est positionné sur un torse du patient.

Un autre objet de l'invention concerne un procédé pour l'amélioration de la précision d'une image biomagnétique d'un patient ledit procédé comprenant une étape d'application sur une partie dudit patient d'un équipement selon l'objet précédent. Cet équipement comporte une pluralité de bobines trois axes, chaque bobine trois axes étant adaptée pour émettre un champ magnétique, les champs magnétiques desdites bobines trois axes étant adaptés pour être détectables par un ensemble de magnétomètres à pompage optique lors d'un examen par imagerie biomagnétique. Le procédé comporte également une étape d'examen d'imagerie biomagnétique pour l'obtention de données biomagnétiques, ladite étape d'examen comprenant une étape d'auto-référencement de la position des magnétomètres à pompage optique par rapport auxdites bobines trois axes. En outre, le procédé comporte une étape d'examen par imagerie par résonnance magnétique pour l'obtention de données IRM. Enfin ce procédé comporte une étape de fusion des données biomagnétiques avec les données IRM par mise en correspondance simple des positions communes acquises précisément lors de l'étape d'examen d'imagerie biomagnétique et de l'étape d'examen IRM.

Ceci permet d'obtenir une image médicale combinée de la partie du patient concernée en s'affranchissant des biais de recalage et fusion de l'art antérieur.

Dans un mode de réalisation particulier, l'examen d'imagerie biomagnétique est un examen par magnétoencéphalographie pour l'obtention de données MEG ou un examen par magnétocardiographie pour l'obtention de données MCG.

Dans un mode de réalisation particulier, les magnétomètres à pompage optique sont des magnétomètres à pompage optique de l'hélium.

La présente invention sera mieux comprise à la lecture de la description détaillée de modes de réalisation pris à titre d'exemples nullement limitatifs et illustrés par les dessins annexés sur lesquels :
[Fig 1] la figure 1 est une vue de face d'un visage d'un patient portant l'équipement selon une première application de l'invention ;
[Fig 2] la figure 2 est une vue de profil du visage du patient de la figure 1 ;
[Fig 3] la figure 3 est une vue de face d'un support adapté pour être accroché sur l'équipement de la figure 1 ;
[Fig 4] la figure 4 est une vue de côté du support de la figure 3 ;
[Fig 5] la figure 5 est une vue de dessus du support de la figure 3 ;
[Fig 6] la figure 6 est une vue de face d'un torse d'un patient portant l'équipement selon une seconde application de l'invention.
[Fig 7] la figure 7 est un schéma illustrant les différentes étapes d'un procédé pour l'obtention d'une image médicale d'un patient portant l'équipement des figures 1 à 6.

Sur les différentes figures, les éléments identiques ou similaires portent les mêmes références.

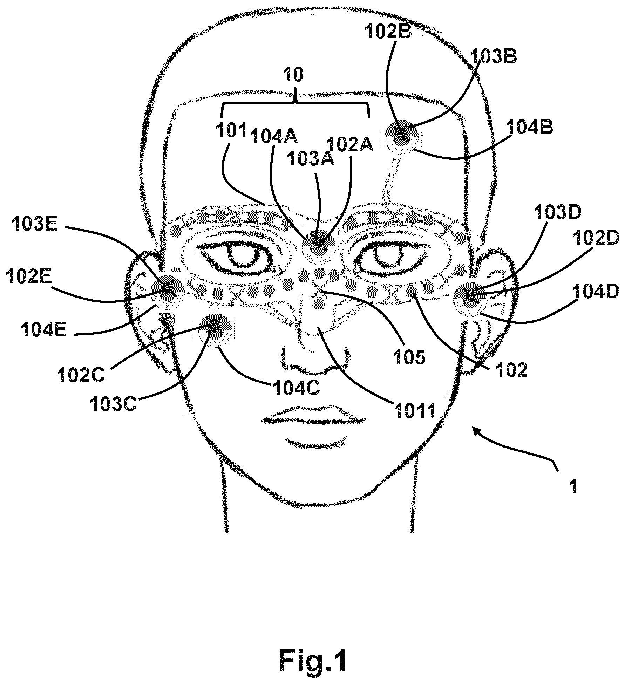

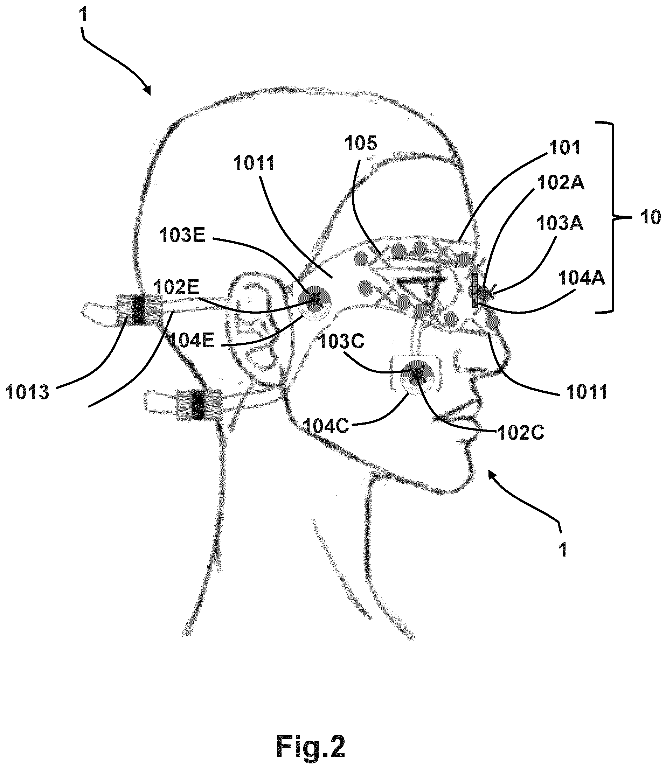

Les figures 1 et 2 représentent un visage d'un patient portant un équipement 10 pour l'obtention d'une image médicale du cerveau du patient. L'équipement 10 comprend :
- une enveloppe 101 ;
- une pluralité de marqueurs 102, 102A, 102B, 102C, 102D, 102E disposés sur l'enveloppe ;
- une pluralité de bobines trois axes 103A, 103B, 103C, 103D, 103E disposées sur l'enveloppe ;
- des supports 104A, 104B, 104C, 104D, 104E pour recevoir tout ou partir des marqueurs 102, 102A, 102B, 102C, 102D, 102E et des bobines trois axes 103A, 103B, 103C, 103D, 103E.

L'enveloppe 101 se présente ici, sous la forme d'un loup adapté pour être positionné sur la partie supérieure du visage 1 du patient autour de ses yeux de façon à couvrir les points singuliers d'intérêt pour l'imagerie MEG. L'enveloppe 101 comprend une partie principale 1011, des lacets élastiques 1012 et un dispositif de réglage 1013. La partie principale 1011 est adaptée pour venir se plaquer sur le visage 1 du patient. Cette partie principale 1011 est configurée pour porter la pluralité de marqueurs 102, 102A, 102B, 102C, 102D, 102E et la pluralité de bobines trois axes 103A, 103B, 103C, 103D, 103E. Les lacets élastiques 1012 sont adaptés pour venir appliquer une contrainte à l'enveloppe afin de l'adapter à la forme du visage du patient. Cela permet de maintenir les marqueurs 102, 102A, 102B, 102C, 102D, 102E et la pluralité de bobines trois axes 103A, 103B, 103C, 103D, 103E au plus proche des saillies osseuses du visage (arcades orbitales, arcades zygomatiques, etc...). En outre, la partie principale 1011 de l'enveloppe 101 couvrant le nez est tendue par les lacets élastiques 1012. Dans un mode de réalisation particulier, l'enveloppe 101 comprend deux lacets élastiques 1012 qui permet le serrage de l'enveloppe 101 à l'arrière de la tête du patient. Un premier lacet élastique passe, par exemple, au-dessus d'une oreille et le second lacet élastique passe au-dessous de l'autre oreille. Le dispositif de réglage 1013 est adapté pour régler et maintenir les lacets élastiques 1012 plaqués à l'arrière de la tête. Il permet d'améliorer le confort du patient lors de l'examen par imagerie par résonnance magnétique, celui-ci étant dans une position allongée pour cet examen. Ce dispositif de réglage 1013 est, par exemple, du même type qu'un dispositif de réglage classique utilisé pour un sac à dos. En variante, les lacets 1012 ne sont pas élastiques. Ce sont des cordons simples que l'on ajuste grâce au dispositif de réglage 1013.

La partie principale 1011 et les lacets élastiques 1012 de l'enveloppe 101 sont réalisés dans un matériau en silicone souple, par exemple en silicone 50 shore translucide talqué. Ce matériau est notamment biocompatible et compatible IRM.

Dans un mode de réalisation alternatif, l'enveloppe 101 est de type masque de plongée.

Les marqueurs 102, 102A, 102B, 102C, 102D, 102E sont adaptés pour créer un contraste lors d'obtention d'images du visage du patient portant l'enveloppe 101, au cours d'une opération d'imagerie par résonnance magnétique. Ces marqueurs 102, 102A, 102B, 102C, 102D, 102E ont, ici, la forme d'une pastille présentant un diamètre inférieur ou égal à 6 mm. Ces pastilles sont, par exemple, des pastilles de type MRI pin point ref 187 du fournisseur Beekley Medical ^{®}. Dans un mode de réalisation particulier, les marqueurs 102, 102A, 102B, 102C, 102D, 102E contiennent du gadolinium. En variante, les marqueurs 102, 102A, 102B, 102C, 102D, 102E contiennent en complément du gadolinium ou en remplacement de celui-ci une pluralité de nanoparticules superparamagnétiques. Par « nanoparticules superparamagnétiques », on entend un matériau ferromagnétique ou ferrimagnétique présentant des grains de dimensions nanométriques. Sur l'exemple des figures 1 et 2, l'équipement comporte plus d'une vingtaine de marqueurs 102, 102A, 102B, 102C, 102D, 102E régulièrement répartis sur la partie principale 1011 de l'enveloppe 101. Plus particulièrement, les marqueurs 102, 102A, 102B, 102C, 102D, 102E sont présents sur les arcades orbitaires, les arcades zygomatiques et une aile osseuse du nez. Cela permet d'affiner la localisation du masque sur les images IRM.

Les bobines trois axes 103A, 103B, 103C, 103D, 103E sont adaptées pour émettre un champ magnétique. Par « bobine trois axes », on entend une bobine adaptée pour émettre des flux magnétiques selon trois axes perpendiculaires entre eux. Une telle bobine est, par exemple, une bobine de Helmholtz. Les bobines trois axes 103A, 103B, 103C, 103D, 103E sont ici de faibles dimensions afin d'être portées par l'enveloppe 101. Elles sont représentées par des croix sur la figure 1. Le champ magnétique émis par les bobines trois axes est détectable par un ensemble de magnétomètres à pompage optique lors d'un examen par magnétoencéphalographie. Les magnétomètres à pompage optique se présentent sous la forme de capsules de petites tailles qui sont organisées sous la forme d'un casque (non représenté sur les figures 1 et 2) adapté à la partie supérieure de la tête du patient. Un tel casque peut comprendre 20 à 200 magnétomètres régulièrement répartis. Les magnétomètres à pompage optique sont aptes à mesurer l'intensité et/ou la direction d'un champ magnétique. Chaque magnétomètre comprend un élément interne sensible à un tel champ magnétique. Cet élément interne associé à un dispositif électronique permet d'extraire la mesure du champ magnétique.

Dans le mode de réalisation des figures 1 et 2, l'enveloppe 101 comprend cinq bobines trois axes référencées 103A, 103B, 103C, 103D, 103E. Ainsi, une première bobine trois axes 103A est placée sur le haut du nez du patient. Une seconde bobine trois axes 103B est disposée sur le front du patient. Une troisième bobine trois axes 103C est placée au niveau de la joue droite de ce patient. La quatrième bobine trois axes 103D et la cinquième bobine trois axes 103E sont placées au niveau des points pré-auriculaires (en avant des oreilles) gauche et droite. La seconde bobine trois axes 103B et la troisième bobine trois axes 103C permettent d'améliorer la précision de recalage des magnétomètres à pompage optique. De plus, la troisième bobine trois axes 103C permet en imagerie par résonnance magnétique de repérer sans erreur la droite de la gauche, quel que soit la convention radiologique ou neurologique utilisée pour visualiser les images IRM. D'autres bobines trois axes 103 sont présentes sur d'autres points singuliers du visage du patient, tels que sur des arcades orbitaires, des arcades zygomatiques ou l'aile du nez. Ces bobines trois axes supplémentaires permettent d'améliorer davantage la précision dans le recalage des données MEG et IRM obtenues. Dans un mode de réalisation particulier, l'équipement 10 comprend au moins cinq bobines trois axes.

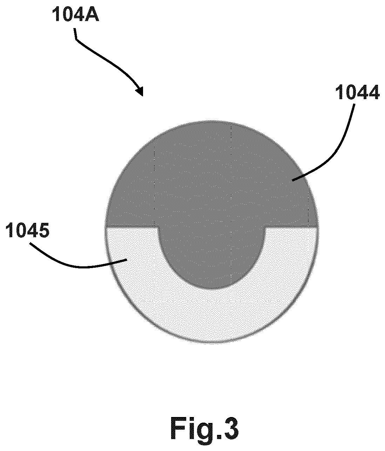

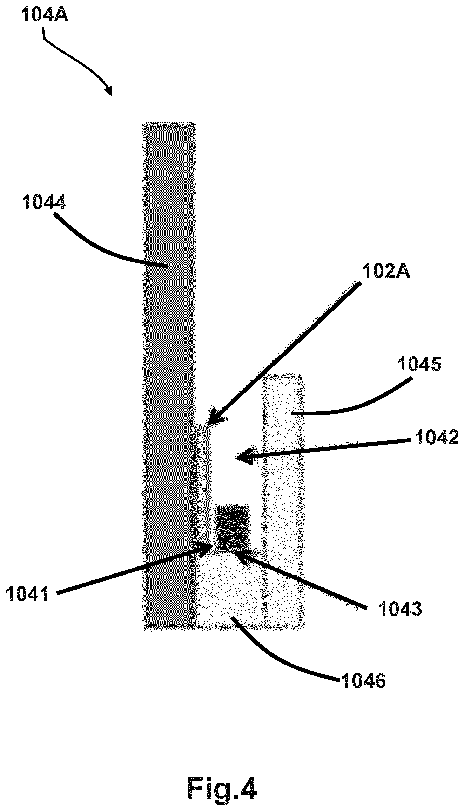

Les supports 104A, 104B, 104C, 104D, 104E sont adaptés pour recevoir des marqueurs 102A, 102B, 102C, 102D, 102E et des bobines trois axes 103A, 103B, 103C, 104D, 104E. A titre d'exemple le support 104A est notamment représenté aux figures 3 à 5. Ce support 104A, comporte :
- une partie arrière 1044 ;
- une partie avant 1045 ;
- une partie intermédiaire 1046.

La partie arrière 1044 est adaptée pour s'accrocher à l'enveloppe 101 afin d'y maintenir le support 104. Cette partie arrière 1044 est un disque présentant un diamètre supérieur à 6 mm.

La partie avant 1045 est adaptée pour maintenir une des bobines trois axes dans le support 104A. Cette partie avant 1045 présente une forme en demi-anneau de diamètre identique à la partie arrière 1044. Cette forme en demi-anneau permet de maintenir le marqueur et la bobine trois axes présents dans le support 104A.

La partie intermédiaire 1046 est disposée entre la partie arrière 1044 et la partie avant 1045 du support 104A.

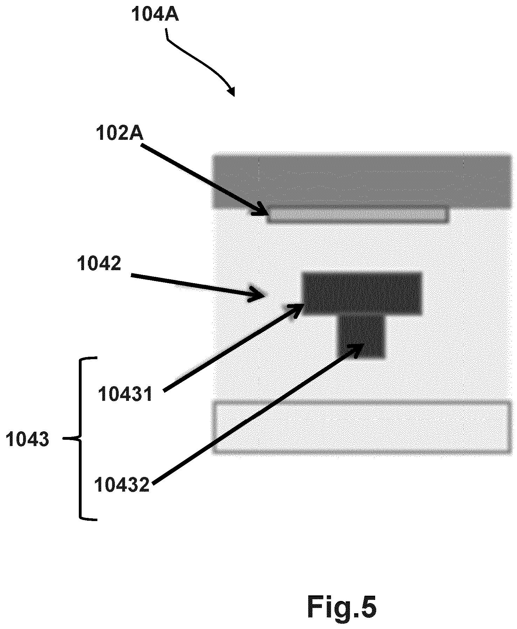

La partie arrière 1044, la partie avant 1045 et la partie intermédiaire 1046 définissent les espaces dans lesquels vont se loger la bobine trois axes et le support associé. Ainsi, le support 104A comporte une première zone de réception 1041 et une seconde zone de réception 1042. La première zone de réception 1041 est adaptée pour recevoir un marqueur 102A. Cette première zone de réception 1041 est délimitée par la partie arrière 1044 du support 104A et par une protubérance 1043. La seconde zone de réception 1042 est délimitée par le marqueur 102A et par la partie avant 1045. La protubérance 1043 est adaptée pour venir se loger dans une cavité associée dans la bobine trois axes. Sur la figure 5, la protubérance 1043 comprend un corps principal 10431 et un pion 10432. Le corps principal 10431 permet de fixer la bobine trois axes dans le support tout en évitant toute rotation de celle-ci dans ledit support. Le pion 10432 s'étend à partir du corps principal 10431. Il a pour rôle d'éviter toute erreur dans le placement de la bobine trois axes au sein du support 104, ce qui permet de placer les axes de la bobine trois axes toujours de la même façon. La description ci-dessus s'applique également pour les autres supports 104B, 104C, 104D, 104E.

Sur l'exemple de la figure 1, l'équipement 10 comprend cinq supports 104A, 104B, 104C, 104D, 104E. En variante, l'équipement 10 comprend plus de cinq supports. Ces autres supports sont par exemple positionnés au niveau des croix 105 de l'équipement 10 visibles aux figures 1 et 2.

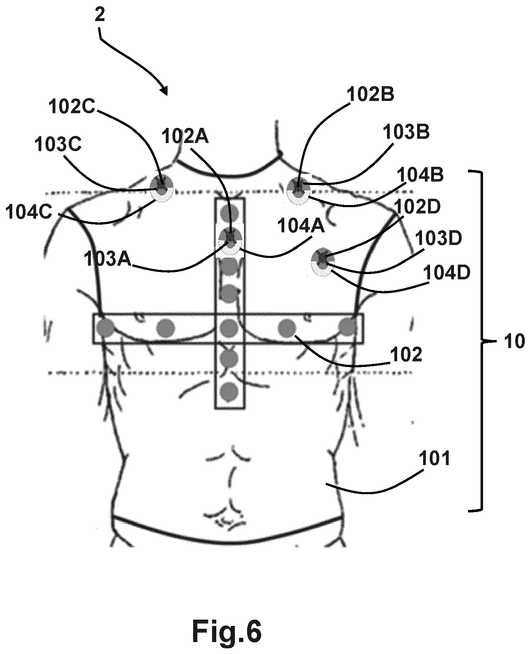

La figure 6 illustre le torse 2 du patient portant l'équipement 10 dans une seconde application de l'invention. Dans ce mode de réalisation, l'équipement 10 comprend une enveloppe 101 se présentant sous la forme d'un gilet thoracique pour l'imagerie biomagnétique du cœur avec localisation des foyers à l'origine des arythmies cardiaques. Dans cette seconde application de l'invention, il s'agit de recaler les données de magnétocardiographie avec les données IRM 3D du cœur. Pour cela, des marqueurs 102 sont régulièrement répartis sur l'enveloppe 101. Ces marqueurs 102 forment une croix dont le centre est situé entre les deux pectoraux du patient. Sur une partie supérieure de cette croix, plus précisément en haut du sternum du patient, l'enveloppe comprend un premier support 104A. Ce support 104A est adapté pour porter un marqueur 102A ainsi qu'une bobine trois axes 103A. D'autres supports 104B, 104C sont également positionnés sur les clavicules du patient. Un autre support 104D est positionné au niveau du pectoral gauche. L'équipement 10 est ici adapté pour obtenir une image combinée du cœur du patient. En vue de détecter les disfonctionnements éventuels de ce cœur, il est possible de réaliser successivement un examen par magnétocardiographie pour l'obtention de données MCG et un examen par imagerie par résonnance magnétique pour l'obtention de données IRM.

On notera que dans cette seconde application de l'invention, il n'est pas nécessaire d'équiper le patient avec un casque portant des magnétomètres à pompage optique. Ces magnétomètres à pompage optique seront directement portés par le gilet thoracique.

On notera également que le dos du patient peut comprendre une pluralité de supports portant des marqueurs et des bobines trois axes pour effectuer les examens MCG et IRM afin d'améliorer la précision de l'imagerie.

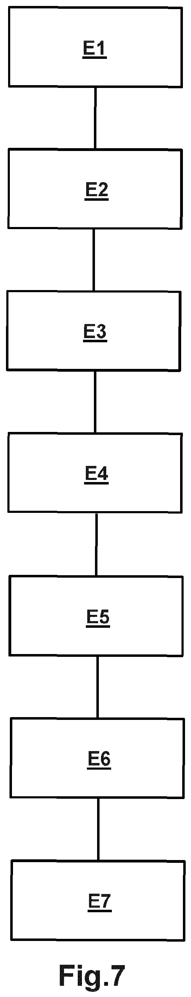

La figure 7 illustre les différentes étapes d'un procédé pour l'amélioration de la précision d'une image biomagnétique du patient portant l'équipement des figures 1 à 2 pour une magnétoencéphalograhie ou l'équipement de la figure 6 pour une magnétocardiographie.

Dans une première étape E1, l'équipement 10 est positionné sur le patient. Si l'on souhaite obtenir une information sur le cerveau, cet équipement 10 est positionné sur le visage 1 du patient. En variante, si c'est le cœur qui fait l'objet de l'examen, l'équipement 10 est positionné sur le torse 2 du patient.

Dans une seconde étape E2, un casque comportant les magnétomètres à pompage optique est positionné sur la tête du patient. Dans le cas d'une magnétocardiographie, les magnétomètres à pompage optique sont positionnés sur le torse du patient sous la forme d'une matrice.

Dans une troisième étape E3, les bobines trois axes 103A, 103B, 103C de l'équipement 10 sont activées.

Dans une quatrième étape E4, les positions des magnétomètres à pompage optique sont déterminées par rapport aux bobines trois axes. Ces positions sont déterminées sans aucun système externe. Comme les bobines trois axes sont présentes au niveau de points singuliers du patient, les magnétomètres à pompage optique sont alors positionnés automatiquement par rapport à ces points singuliers. L'auto-localisation de la position des magnétomètres à pompage optique par rapport à la position de ces bobines trois axes est ainsi effectuée. On notera que dans un mode préférentiel de l'invention, les magnétomètres sont des magnétomètres à pompage optique de l'hélium.

Dans une cinquième étape E5, les données MEG ou les données MCG sont acquises au cours de l'examen par magnétoencéphalographie ou de l'examen par magnétocardiographie. Ces flux de données sont enregistrés lors de plusieurs séquences d'enregistrement.

Dans une sixième étape E6, une étape d'examen par imagerie par résonnance magnétique est réalisée pour obtenir des données IRM (bobine trois axes sont préalablement retirées des supports). Les marqueurs disposés sur l'équipement 10 restent en place et permettent le repérage des mêmes points singuliers que ceux localisées par l'examen MEG ou l'examen MCG à l'aide des bobines.

Dans une septième étape E7, les données MEG ou les données MCG et les données IRM sont traitées pour être fusionnées par mise en correspondance des coordonnées de points singuliers communs acquis dans les deux modalités. Il est alors possible d'obtenir une image combinée exploitable par un praticien. Cette image combinée permet de visualiser les variations de champs électriques reconstruits à partir de la magnétoencéphalographie ou de la magnétocardiographie dans le volume généré par l'examen par imagerie par résonnance magnétique.

L'équipement objet de l'invention apporte les avantages suivants :
- il permet de proposer un support de type masque ou gilet permettant de positionner, dans des supports communs, des bobines trois axes émettrices et des pastilles de gadolinium visibles sur les images IRM ;
- il permet d'exploiter la mesure du champ magnétique émis par les bobines trois axes, par les magnétomètres à pompage optique présents sur la tête ou sur le thorax du patient ;
- il permet d'exploiter la co-localisation des bobines trois axes visibles en MEG ou en MCG et des pastilles de gadolinium visibles en IRM, du fait de leur support commun, pour le recalage des données MEG/IRM ou MCG/IRM. L'utilisation de supports communs pour les bobines émettrices et les pastilles de gadolinium assure un recalage optimal entre les données MEG/MCG et IRM puisque les mêmes points seront exactement localisés avec les deux techniques ;
- la mesure du champ magnétique émis par les bobines trois axes permet une auto-localisation de chaque capteur à magnétomètre à pompage optique de l'hélium ;
- l'utilisation d'un masque ajusté sur le visage du patient ou d'un gilet sur le thorax du patient, utilisable à la fois en MEG/MCG (avec les bobines trois axes permettant une localisation) et en IRM (avec les pastilles de gadolinium) limite fortement les sources de biais lors du recalage MEG/MCG avec l'IRM puisque la position des bobines et des pastilles de gadolinium est identique ;
- l'équipement ne requiert pas de système de numérisation externe. Les magnétomètres à pompage optique de l'hélium présents sur la tête du patient permettent de mesurer le champ magnétique émis par les bobines trois axes et de les localiser. Les pastilles de gadolinium sont visualisées directement sur les images IRM ;
- le patient porte son masque ou son gilet thoracique durant les deux types d'enregistrement MEG/IRM ou MCG/IRM.

L'invention n'est pas limitée aux modes de réalisation et variantes présentées et d'autres modes de réalisation et variantes apparaîtront clairement à l'homme du métier.

Ainsi, différentes tailles d'équipement sont envisagées de façon à s'adapter à toutes les tranches d'âge (bébé, enfant, adulte).

Ainsi, les dimensions de supports peuvent varier selon le type de bobine trois axes et selon la taille des marqueurs.

## Revendications

1. Equipement pour l'amélioration de la précision d'une image biomagnétique d'un patient, ledit équipement (10) comportant :
- une enveloppe (101) adaptée pour être positionnée sur une partie anatomique du patient ;
- une pluralité de marqueurs (102, 102A, 102B, 102C, 102D, 102E) disposés sur l'enveloppe (101), lesdits marqueurs (102, 102A, 102B, 102C, 102D, 102E) étant adaptés pour créer un contraste lors d'une obtention d'une image du patient réalisée par imagerie par résonnance magnétique ;
- au moins cinq bobines trois axes (103A, 103B, 103C, 103D, 103E), chaque bobine trois axes étant adaptée pour émettre un champ magnétique, les champs magnétiques desdites bobines trois axes (103A, 103B, 103C, 103D, 103E) étant détectables par un ensemble de magnétomètres à pompage optique lors d'un examen par imagerie biomagnétique (MEG, MCG), lesdites bobine trois axes (103A, 103B, 103C, 103D, 103E) étant disposées sur l'enveloppe (101) de sorte que lorsque l'équipement (10) est positionné sur le patient, chaque bobine trois axes (103A, 103B, 103C, 103D, 103E) est localisée sur un point singulier du patient ; les bobines trois axes (103A, 103B, 103C, 103D, 103E) et des marqueurs (102A, 102B, 102C, 102D, 102E) de la pluralité de marqueurs (102, 102A, 102B, 102C, 102D, 102E) sont placés au même endroit sur l'enveloppe de sorte que lorsque ladite enveloppe (101) est positionnée sur le patient, des points singuliers de la partie du patient sont détectables par imagerie par résonnance magnétique et par imagerie biomagnétique (MEG, MCG), dans lequel ledit équipement (10) comporte des supports (104A, 104B, 104C, 104D, 104E), chaque support (104A, 104B, 104C, 104D, 104E) ayant une première zone de réception (1041) adaptée pour recevoir un des marqueurs (102A, 102B, 102C, 102D, 102E) et une seconde zone de réception (1042) adaptée pour recevoir une des bobines trois axes (103A, 103B, 103C, 103D, 103E), ledit marqueur et ladite bobine trois axes étant alors placés au même endroit sur l'enveloppe (101), dans lequel la seconde zone de réception (1042) comprend une protubérance (1043) adaptée pour venir se loger dans une cavité associée de la bobine trois axes (103A, 103B, 103C, 103D, 103E) **caractérisé en ce que** la protubérance (1043) comprend un corps principal (10431) et un pion (10432) s'étendant à partir dudit corps principal (10431), ladite protubérance (1043) ayant un rôle de détrompeur pour le positionnement de la bobine trois axes (103A, 103B, 103C, 103D, 103E) dans le support (104A, 104B, 104C, 104D, 104E).

2. Equipement selon la revendication 1, dans lequel l'enveloppe (101) est réalisée dans un matériau en silicone biocompatible souple.

3. Equipement selon l'une quelconque des revendications 1 à 2, dans lequel les marqueurs (102, 102A, 102B, 102C, 102D, 102E) comprennent du gadolinium et/ou une pluralité de nanoparticules superparamagnétiques.

4. Equipement selon l'une quelconque des revendications 1 à 3, dans lequel chaque marqueur (102, 102A, 102B, 102C, 102D, 102E) a la forme d'une pastille présentant un diamètre inférieur ou égal à 6 mm.

5. Equipement selon l'une quelconque des revendications 1 à 4, dans lequel ledit équipement (10) est adapté pour être positionné sur un visage (1) du patient ou dans lequel ledit équipement (10) est adapté pour être positionné sur un torse (2) du patient.

6. Procédé pour l'amélioration de la précision d'une image biomagnétique d'un patient ledit procédé comprenant :
- une étape (E1) d'application sur une partie dudit patient d'un équipement (10) selon l'une quelconque des revendications 1 à 5, ledit équipement (10) comportant une pluralité de bobines trois axes (103A, 103B, 103C, 103D, 103E), chaque bobine trois axes (103A, 103B, 103C, 103D, 103E) étant adaptée pour émettre un champ magnétique, les champs magnétiques desdites bobines trois axes étant détectables par un ensemble de magnétomètres à pompage optique lors d'un examen d'imagerie biomagnétique (MEG, MCG) ;
- une étape (E4) d'auto-référencement de la position des magnétomètres à pompage optique par rapport auxdites bobines trois axes (103A, 103B, 103C, 103D, 103E) ;
- une étape (E5) d'examen par imagerie biomagnétique (MEG, MCG) pour l'obtention de données biomagnétiques (données MEG, données MCG) ;
- une étape (E6) d'examen par imagerie par résonnance magnétique pour l'obtention de données IRM ;
- une étape (E7) de fusion des données biomagnétique (données MEG, données MCG) avec les données IRM par mise en correspondance simple de positions communes acquises précisément lors de l'étape d'examen d'imagerie biomagnétique et de l'étape d'examen IRM.

7. Procédé selon la revendication 6, dans lequel l'examen d'imagerie biomagnétique est un examen par magnétoencéphalographie pour l'obtention de données MEG ou un examen par magnétocardiographie pour l'obtention de données MCG.

8. Procédé selon selon l'une quelconque des revendications 6 ou 7, dans lequel les magnétomètres à pompage optique sont des magnétomètres à pompage optique de l'hélium.

## Patentansprüche

1. Vorrichtung zur Verbesserung der Präzision eines biomagnetischen Bilds von einem Patienten, wobei die Vorrichtung (10) umfasst:
- eine Hülle (101), angepasst, um auf einen anatomischen Bereich des Patienten positioniert zu werden;
- eine Vielzahl von Markern (102, 102A, 102B, 102C, 102D, 102E), angeordnet auf der Hülle (101), wobei die Marker (102, 102A, 102B, 102C, 102D, 102E) angepasst sind, um bei dem Erhalten eine Bilds des Patienten, das durch Magnetresonanzbildgebung durchgeführt wird, einen Kontrast zu erzeugen;
- mindestens fünf dreiachsige Spulen (103A, 103B, 103C, 103D, 103E), wobei jede dreiachsige Spule zum Emittieren eines Magnetfelds angepasst ist, wobei die Magnetfelder der dreiachsigen Spulen (103A, 103B, 103C, 103D, 103E) bei einer Untersuchung durch biomagnetische Bildgebung (MEG, MCG) durch eine optisch gepumpte Magnetometerbaugruppe detektierbar sind, wobei die dreiachsigen Spulen (103A, 103B, 103C, 103D, 103E) derart auf der Hülle (101) angeordnet sind, dass, wenn die Vorrichtung (10) auf dem Patienten positioniert wird, jede dreiachsige Spule (103A, 103B, 103C, 103D, 103E) sich an einem einzelnen Punkt des Patienten befindet; wobei die dreiachsigen Spulen (103A, 103B, 103C, 103D, 103E) und die Marker (102A, 102B, 102C, 102D, 102E) der Vielzahl von Markern (102, 102A, 102B, 102C, 102D, 102E) derart an der gleichen Stelle auf der Hülle platziert sind, dass, wenn die Hülle (101) auf dem Patienten positioniert ist, die einzelnen Punkte des Bereichs des Patienten durch Magnetresonanzbildgebung und durch biomagnetische Bildgebung (MEG, MCG) detektierbar sind, wobei die Vorrichtung Träger (104A, 104B, 104C, 104D, 104E) umfasst, wobei jeder Träger (104A, 104B, 104C, 104D, 104E) eine erste Aufnahmezone (1041), angepasst zum Aufnehmen eines der Marker (102A, 102B, 102C, 102D, 102E), und eine zweite Aufnahmezone (1042) aufweist, angepasst zum Aufnehmen einer der dreiachsigen Spulen (103A, 103B, 103C, 103D, 103E), wobei der Marker und die dreiachsige Spule somit an der gleichen Stelle auf der Hülle (101) platziert sind, wobei die zweite Aufnahmezone (1042) einen Vorsprung (1043) beinhaltet, angepasst zur Unterbringung in einem mit den dreiachsigen Spulen (103A, 103B, 103C, 103D, 103E) assoziierten Hohlraum, **dadurch gekennzeichnet, dass** der Vorsprung (1043) einen Hauptkörper (10431) und einen Stift (10432) beinhaltet, der sich ausgehend von dem Hauptkörper (10431) erstreckt, wobei der Vorsprung (1043) für die Positionierung der dreiachsigen Spule (103A, 103B, 103C, 103D, 103E) in dem Träger (104A, 104B, 104C, 104D, 104E) eine Rolle als Verwechslungsschutz aufweist.

2. Vorrichtung nach Anspruch 1, wobei die Hülle (101) aus einem weichen, biokompatiblen Material aus Silikon hergestellt ist.

3. Vorrichtung nach einem der Ansprüche 1 bis 2, wobei die Marker (102, 102A, 102B, 102C, 102D, 102E) Gadolinium und/oder eine Vielzahl von superparamagnetischen Nanopartikeln beinhalten.

4. Vorrichtung nach einem der Ansprüche 1 bis 3, wobei jeder Marker (102, 102A, 102B, 102C, 102D, 102E) die Form einer Pastille aufweist, die einen Durchmesser kleiner als oder gleich 6 mm aufweist.

5. Vorrichtung nach einem der Ansprüche 1 bis 4, wobei die Vorrichtung (10) angepasst ist, um auf einem Gesicht (1) des Patienten positioniert zu werden, oder wobei die Vorrichtung (10) angepasst ist, um auf einem Torso (2) des Patienten positioniert zu werden.

6. Verfahren zur Verbesserung der Präzision eines biomagnetischen Bilds von einem Patienten, wobei das Verfahren beinhaltet:
- einen Schritt (E1) der Anwendung, auf einem Bereich des Patienten, einer Vorrichtung (10) nach einem der Ansprüche 1 bis 5, wobei die Vorrichtung (10) eine Vielzahl von dreiachsigen Spulen (103A, 103B, 103C, 103D, 103E) umfasst, wobei jede dreiachsige Spule (103A, 103B, 103C, 103D, 103E) angepasst ist zum Emittieren eines Magnetfelds, wobei die Magnetfelder der dreiachsigen Spulen bei einer biomagnetischen Bildgebungsuntersuchung (MEG, MCG) durch eine optisch gepumpte Magnetometerbaugruppe detektierbar sind;
- einen Schritt (E4) der Selbstreferenzierung der Position der optisch gepumpten Magnetometer in Bezug auf die dreiachsigen Spulen (103A, 103B, 103C, 103D, 103E);
- einen Schritt (E5) der Untersuchung durch biomagnetische Bildgebung (MEG, MCG) zum Erhalten biomagnetischer Daten (MEG-Daten, MCG-Daten);
- einen Schritt (E6) der Untersuchung durch Magnetresonanzbildgebung zum Erhalten von MRI-Daten;
- einen Schritt (E7) der Fusion von biomagnetischen Daten (MEG-Daten, MCG-Daten) mit den MRI-Daten durch einfache Zuordnung der gemeinsamen Positionen, die bei dem Schritt der biomagnetischen Bildgebung und dem Schritt der MRI-Untersuchung präzise erfasst wurden.

7. Verfahren nach Anspruch 6, wobei die biomagnetische Untersuchung eine Untersuchung durch Magnetoenzephalographie zum Erhalten von MEG-Daten oder eine Untersuchung durch Magnetokardiographie zum Erhalten von MCG-Daten ist.

8. Verfahren nach einem der Ansprüche 6 oder 7, wobei die optisch gepumpten Magnetometer optisch gepumpte Helium-Magnetometer sind.

## Claims

1. Device for improving the precision of a biomagnetic image of a patient, said device (10) comprising:
- a covering (101) suitable for being positioned on an anatomical part of the patient;
- a plurality of markers (102, 102A, 102B, 102C, 102D, 102E) disposed on the covering (101), said markers (102, 102A, 102B, 102C, 102D, 102E) being suitable for creating a contrast during an obtaining of an image of the patient, done by magnetic resonance imaging;
- at least five three-axis coils (103A, 103B, 103C, 103D, 103E), each three-axis coil being suitable for emitting a magnetic field, the magnetic fields of said three-axis coils (103A, 103B, 103C, 103D, 103E) being able to be detected by a set of optical pumping magnetometers during an examination by biomagnetic imaging (MEG, MCG), said three-axis coils (103A, 103B, 103C, 103D, 103E) being disposed on the covering (101), such that when the device (10) is positioned on the patient, each three-axis coil (103A, 103B, 103C, 103D, 103E) is located on a singular point of the patient, the three-axis coils (103A, 103B, 103C, 103D, 103E) and the markers (102A, 102B, 102C, 102D, 102E) of the plurality of markers (102, 102A, 102B, 102C, 102D, 102E) are placed at the same location on the covering so that, when said covering (101) is positioned on the patient, singular points of the part of the patient can be detected by magnetic resonance imaging and by biomagnetic imaging (MEG, MCG), in which said device (10) comprises supports (104A, 104B, 104C, 104D, 104E), each support (104A, 104B, 104C, 104D, 104E) having a first receiving zone (1041), suitable for receiving one of the markers (102A, 102B, 102C, 102D, 102E), and a second receiving zone (1042), suitable for receiving one of the three-axis coils (103A, 103B, 103C, 103D, 103E), said marker and said three-axis coil thus being placed at the same location on the covering (101), in which the second receiving zone (1042) comprises a protrusion (1043), suitable for being housed in an associated cavity of the three-axis coil (103A, 103B, 103C, 103D, 103E), **characterised in that** the protrusion (1043) comprises a main body (10431) and a pin (10432) extending from said main body (10431), said protrusion (1043) having a locking tab role, for the positioning of the three-axis coil (103A, 103B, 103C, 103D, 103E) in the support (104A, 104B, 104C, 104D, 104E).

2. Device according to claim 1, wherein the covering (101) is made of a flexible biocompatible silicone material.

3. Device according to any one of claims 1 to 2, wherein the markers (102, 102A, 102B, 102C, 102D, 102E) comprise gadolinium and/or a plurality of superparamagnetic nanoparticles.

4. Device according to any one of claims 1 to 3, wherein each marker (102, 102A, 102B, 102C, 102D, 102E) has the shape of a dot having a diameter less than or equal to 6mm.

5. Device according to any one of claims 1 to 4, wherein said device (10) is suitable for being positioned on a face (1) of the patient, or wherein said device (10) is suitable for being positioned on a torso (2) of the patient.

6. Method for improving the precision of a biomagnetic image of a patient, said method comprising:
- a step (E1) of applying a device (10) according to any one of claims 1 to 5 on a part of said patient, said device (10) comprising a plurality of three-axis coils (103A, 103B, 103C, 103D, 103E), each three-axis coil (103A, 103B, 103C, 103D, 103E) being suitable for emitting a magnetic field, the magnetic fields of said three-axis coils being able to be detected by a set of optical pumping magnetometers during a biomagnetic imaging examination (MEG, MCG);
- a step (E4) of self-referencing the position of the optical pumping magnetometers with respect to said three-axis coils (103A, 103B, 103C, 103D, 103E);
- a step (E5) of examining, by biomagnetic imaging (MEG, MCG), for obtaining biomagnetic data (MEG data, MCG data);
- a step (E6) of examining, by magnetic resonance imaging, for obtaining MRI data;
- a step (E7) of merging biomagnetic data (MEG data, MCG data) with MRI data by simple matching of common positions precisely acquired during the biomagnetic imaging examination step and the MRI examination step.

7. Method according to claim 6, wherein the biomagnetic imaging examination is a magnetoencephalography examination for obtaining MEG data or a magnetocardiography examination for obtaining MCG data.

8. Method according to any one of claims 6 or 7, wherein the optical pumping magnetometers are helium optical pumping magnetometers.
